(19) Europäisches Patentamt
European Patent Office
Office européen des brevets

(11) **EP 1 917 993 B1**

(12) **EUROPEAN PATENT SPECIFICATION**

(45) Date of publication and mention
of the grant of the patent:
**07.11.2012 Bulletin 2012/45**

(21) Application number: **06757193.5**

(22) Date of filing: **08.06.2006**

(51) Int Cl.:
*A61M 16/06* (2006.01)     *A61F 5/08* (2006.01)

(86) International application number:
**PCT/JP2006/311557**

(87) International publication number:
**WO 2007/023607 (01.03.2007 Gazette 2007/09)**

(54) **NOSTRIL PLUG FOR IMPROVING ARTICULATORY DISORDER**

NASENLOCH-STOPFEN ZUR BESSERUNG VON ARTIKULATIONSSTÖRUNGEN

BOUCHE-NARINE DESTINÉ À REDUIRE LES TROUBLES ARTHRIQUES

(84) Designated Contracting States:
**DE**

(30) Priority: **26.08.2005 JP 2005246737**

(43) Date of publication of application:
**07.05.2008 Bulletin 2008/19**

(73) Proprietor: **National University Corporation Okayama University**
**Okayama-shi, Okayama 700-8530 (JP)**

(72) Inventor: **MINAGI, Shogo**
**Okayama-shi**
**Okayama 700-8558 (JP)**

(74) Representative: **HOFFMANN EITLE**
**Patent- und Rechtsanwälte**
**Arabellastrasse 4**
**81925 München (DE)**

(56) References cited:
**WO-A-2005/004993     JP-A- 49 087 191**
**JP-A- 2001 526 577     JP-U- 03 059 270**
**US-A- 3 884 223     US-A- 5 425 359**
**US-A- 6 119 690     US-A1- 2004 255 947**

**Description**

Technical Field

The present invention relates to a nostril plug for reducing functional articulatory disorder by suppressing the leaking of exhalation from a nose by the insertion of the nostril plug into the inside of nostril.

Background of the Invention

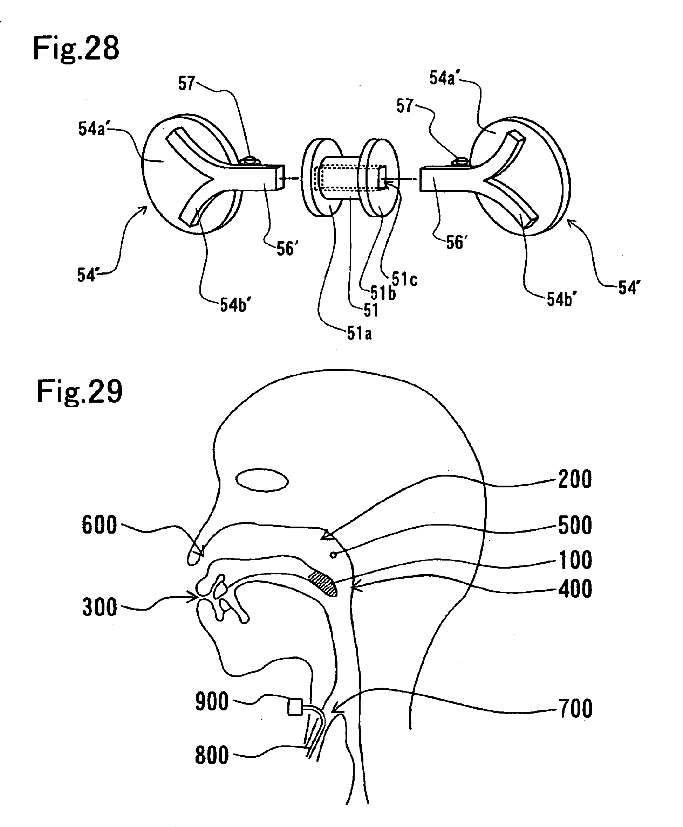

Usually, when a person utters a sound, air vibrations corresponding to vibrations generated in exhalation by vocal cords are generated, and the air vibrations are discharged from a mouth with the exhalation. Here, as can be understood from Fig. 29 which is an explanatory view of the structure in the vicinity of a nose and a throat of a person, a velum 100 prevents the exhalation from being leaked to a nasal-cavity-200 side and hence, the exhalation is mainly discharged from the mouth 300 thus allowing the person to utter sounds clearly.

However, when the closure of the rhinopharynx 400 becomes incomplete due to cerebral infarction, cerebral hemorrhage, myasthenia, congenital anomaly, abnormal growth, external wound or the like, in uttering a sound, a portion of the exhalation is leaked into the nasal-cavity-200 side thus making it difficult for a person to utter sounds clearly. This phenomenon is referred to as functional articulatory disorder.

In such a case, there has been known a technique which can reduce functional articulatory disorder by accelerating the closure of the rhinopharynx 400. Such a technique mainly adopts a method which narrows the rhinopharynx 400 by an operation, a method which mounts a velum lifting device for lifting the velum 100 in a sagging state due to flaccidity of the velopharyngeal muscles, or a method which uses a speech aid arranging a plug behind a velum 100. In Fig. 29, numeral 500 indicates an opening of Eustachian tube and numeral 600 indicates a nostril.

As shown in Fig. 29, when a cannula 800 is mounted in a respiratory tract 700, the exhalation leaks from a portion of the cannula 800 and hence, the discharge of a sufficient quantity of exhalation from a mouth becomes difficult thus causing articulatory disorder. In such a case, a check valve 900 referred to as a speaking valve is mounted in the cannula 800 thus forming a passage of inhaled air from a respiratory organ and preventing passing of exhalation thus discharging the exhalation through a mouth whereby the articulatory disorder is minimally generated. As the check valve 900 used in such a case, a filter-mounted valve, trachea narrow hole valve and the like have been proposed (see patent document 1 and patent document 2, for example).

Further, as a method for reducing articulatory disorder, there has been reported an example where an utter-sound treatment is applied to a patient who is considered to be suffering from articulatory disorder caused by muscle flaccidity attributed to traumatic brain injury particularly. In this method, a nostril plug is tightly inserted into the inside of nostrils 600 to completely stop nasal breathing thus stopping leaking of the exhalation from the nostrils and hence, leaking of the exhalation to the nasal-cavity-200 side can be prevented (see non-patent document 1, for example).

Here, as an instrument used in a state where the instrument is inserted into the nostrils 600, there has been known a nasal plug which is provided for preventing the intrusion of pollens from the nostrils 600 (see patent document 3, for example). This nasal plug is configured to incorporate a porous film made of plastic fibers and a filter made of non-woven fabric therein. The nasal plug can remove fine particles such as pollens while possessing air permeability which does not interrupt breathing. However, the nasal plug is not configured to prevent leaking of the exhalation and hence, the nasal plug cannot reduce the articulatory disorder.

Patent document 1: USP 5259378

Patent document 2: USP 4538607

Patent document 3: JP-A-09-239047

Non-patent document 1: Paper " A Device for the Management of Velopharyngeal Incompetence" written by Stewart DS and Rieger WJ described on pages 149 to 155, No. 2, Vol. 2, Journal of Medical Speech-Language Pathology published in 1994

US patent application US 3,884,223 refers to a filter device for insertion in the nostrils to prevent foreign particles from entering the nasal passages by excluding physically the greater part of such particles and the rest by electrostatic precipitation. A flat closure is provided to close the peripheral air passage between the outside of the cylinder and the inner section of a removably mounted member during inhalation. Further, an air canal has a one way valve which opens upon application of exhaling pressure.

Further prior art is known from document US 2004/0255947 A1 referring to a filtering face mask developed for persons who work in polluted environments.

Disclosure of the Invention

Problems that the Invention is to Solve

Although the functional articulatory disorder can be reduced by an operation which narrows the rhinopharynx or the mounting of the velum lifting device, a physical burden imposed on a physically handicapped person suffering from the functional articulatory disorder is large and hence, there has been a demand for a method which can reduce such a physical burden of the physically handicapped person.

The method which inserts the nostril plug into the nostrils is a method which can reduce the physical burden. However, when the nostril plug is inserted into the nostrils, there arise following drawbacks. That is, breathing through the nose can be completely interrupted. Further, due to a pressure change in the inside of the nasal cavity frequently generated when a patient swallows saliva due to closing of the nostrils by the nostril plug, the patient is liable to easily feel sense of incongruity or pains in ears which are communicated with the nose through eustachians and hence, the patient is liable to easily feel extreme discomfort.

On the other hand, the nasal plug maintaining the air permeability is hardly effective in the reduction of functional articulatory disorder.

In view of the above-mentioned circumstance, the inventor of the present invention has made researches and developments for providing functional-articulatory-disorder reducing equipment which can reduce functional articulatory disorder by preventing leakage of exhalation from nostrils by the closure of the nostrils and, at the same time, can make a patient hardly feel discomfort in use, and has arrived at the present invention. Means for Solving the Problems

The present invention is directed to a nostril plug for suppressing leakage of exhalation from a nose as defined in claim 1.

Further, the present invention is characterized in that the support body is formed of a tubular body inscribed in the nostril, and the valve unit is removably fitted in the support body.

Further, the present invention is characterized in that the valve unit is configured to be separated from the support body by pressure when the pressure inside the nostril reaches a predetermined level or more by exhalation.

Further, the present invention is characterized in that the nostril plug includes a first support body inserted into a left nostril, a second support body inserted into a right nostril, and a connecting bar which connects the first support body and the second support body, and the connecting bar which is arranged at a position where the connecting bar is brought into contact with a philtrum when the first support body is inserted into the left nostril and the second support body is inserted into the right nostril.

Further, the present invention is characterized in that the nostril plug includes a first support body inserted into aleftnostril, a second support body inserted into a right nostril, and a connecting bar which connects the first support body and the second support body, and the valve unit mounted on either one of the support bodies is configured to be more easily separated from the support body than the other valve unit.

Further, the present invention is characterized in that the valve unit includes a valve seat formed with a plurality of ventilation openings and a valve element which closes the ventilation openings.

Further, the present invention is characterized in that the valve unit includes a tubular frame having both ends thereof opened, a planar valve seat which is mounted on one opening end side of the frame and is configured to close the opening end, and a valve element which is mounted on a side surface of the valve seat facing the inside of the frame and is configured to close a ventilation opening formed in the valve seat, on a surface side of the valve seat on which the valve element is arranged, a guide arm having a hook which is engaged with a projecting portion formed on an inner peripheral surface of the frame is arranged along the longitudinal direction of the frame, and the valve seat is configured to be advanced or retracted in the longitudinal direction of the frame by restricting the guide arm with the frame, and in a state that a pressure inside the nostril reaches a predetermined level or more through exhalation, the valve seat is advanced from the frame due to the pressure to form a gap thus releasing the pressure.

Further, the present invention is characterized in that the support body is formed of a rod-shapedbodywhich is configured to be inserted into a through hole formed in a nasal septum, and has one end portion thereof arranged in the inside of the left nostril and the other end portion thereof arranged in the inside of the right nostril, and the valve unit includes a valve seat formed of a resilient body mounted on an end portion of the support body and a valve element which is configured to close a ventilation opening formed in the valve seat, and in a state that a pressure inside the nostril reaches a predetermined level or more through exhalation, the valve seat is configured to be resiliently deformed due to the pressure to form a gap between the valve seat and the nostril thus releasing the pressure.

Advantage of the Invention

According to the nostril plug described in claim 1, the nostril plug nostril plug for suppressing leakage of exhalation from a nose by insertion of the nostril plug into the nostril includes a valve unit for suppressing only passage of exhalation in breathing, and a support body for supporting the valve unit, the support body mounting the valve unit thereon and configured to be arranged in the inside of the nostril.

Further, with the use of the nostril plug according to claim 1, the patient can inhale air from a nose and hence, it is unnecessary for the patient to inhale air through a mouth thus allowing the patient to perform breathing in an approximately natural state. Accordingly, the patient inserting the nostril plug of the present invention can use the nostril plug without feeling discomfort.

Further, according to the nostril plug described in claim 2, in the nostril plug described in claim 1, the support body is formed of a tubular body inscribed in the nostril, and the valve unit is removably fitted in the support body. Due to such a constitution, the support body can be formed in conformity with a shape of the nostril which differs for every individual at a relatively low cost, and can enhance wearing feeling and stability of the support body. Further, by allowing the common use of the valve unit, a manufacturing cost of the support body can be reduced. Still further, when the support body is degraded or the valve unit is degraded, only the support body or the valve unit needs to be exchanged thus realizing the reduction of a maintenance cost.

Further, according to the nostril plug described in claim 3, in the nostril plug described in claim 2, the valve unit is configured to be separated from the support body by pressure when the pressure inside the nostril reaches a predetermined level or more through exhalation. Due to such a constitution, when a pressure inside the nostril is sharply increased due to sneezing or the like, only the valve unit is removed from the support body to alleviate the pressure inside the nostril thus preventing the removal of the support body from the nostril. Accordingly, the valve unit can be easily mounted again.

Further, according to the nostril plug described in claim 4, in the nostril plug described in claim 3, the nostril plug includes the first support body inserted into the left nostril, the second support body inserted into the right nostril, and a connecting bar which connects the first support body and the second support body, and the connecting bar is arranged at a position where the connecting bar is brought into contact with a philtrum when the first support body is inserted into the left nostril and the second support body is inserted into the right nostril. Due to such a constitution, when the nostril plug is inserted into the nostril, it is possible to make the connecting bar inconspicuous. Further, in removing the nostril plug, the nostril plug can be easily removed by making use of the connecting bar as a grip.

Further, according to the nostril plug described in claim 5, in the nostril plug described in claim 3, the nostril plug includes the first support body inserted into the left nostril, the second support body inserted into the right nostril, and a connecting bar which connects the first support body and the second support body, and the valve unit mounted on either one of the support bodies is configured to be more easily separated from the support body than the other valve unit. Due to such a constitution, when a pressure inside the nostril is sharply increased due to sneezing or the like, only the valve unit which is liable to be easily separated is removed from the support body for alleviating the pressure inside the nostril and, thereafter, only one separated valve unit may be mounted again thus facilitating the re-mounting of the valve unit.

Further, according to the nostril plug described in claim 6, in the nostril plug described in any one of claims 1 to 5, the valve unit includes a valve seat formed with a plurality of ventilation openings and a valve element which closes the ventilation openings.

Further, according to the nostril plug described in claim 7, in the nostril plug described in claim 1, the valve unit includes a tubular frame having both ends thereof opened, a planar valve seat which is mounted on one opening end side of the frame and is configured to close the opening end, and a valve element which is mounted on a side surface of the valve seat facing the inside of the frame and is configured to close a ventilation opening formed in the valve seat, on a surface side of the valve seat on which the valve element is arranged, a guide arm having a hook which is engaged with a projecting portion formed on an inner peripheral surface of the frame is arranged along the longitudinal direction of the frame, and the valve seat is configured to be advanced or retracted in the longitudinal direction of the frame by restricting the guide arm with the frame, and in a state that a pressure inside the nostril reaches a predetermined level or more through exhalation, the valve seat is advanced from the frame due to the pressure to form a gap thus releasing the pressure. Due to such a constitution, when a pressure inside the nostril is sharply increased due to sneezing or the like, the pressure can be released while preventing the removal of the valve unit.

Further, according to the nostril plug described in claim 8, in the nostril plug described in claim 1, the support body is formed of a rod-shaped body which is configured to be inserted into a hole piercing the nasal septum, and has one end portion thereof arranged in the inside of the left nostril and the other end portion thereof arranged in the inside of the right nostril, and the valve unit includes a valve seat formed of a resilient body mounted on an end portion of the support body and a valve element which is configured to close a ventilation opening formed in the valve seat, and in a state that a pressure inside the nostril reaches a predetermined level or more through exhalation, the valve seat is configured to be resiliently deformed due to the pressure to form a gap between the valve seat and the nostril thus releasing the pressure. Due to such a constitution, the valve unit and the support body can be miniaturized thus largely reducing discomfort attributed to insertion of the nostril plug.

BRIEF Description of the Drawings

Fig. 1 is a perspective view of a nostril plug of a first embodiment as viewed from an oblique front position.
Fig. 2 is a perspective view of the nostril plug of the first embodiment as viewed from an oblique rear position.
Fig. 3 is a front view of the nostril plug of the first embodiment.
Fig. 4 is a back view of the nostril plug of the first embodiment.
Fig. 5 is a cross-sectional view of the nostril plug of the first embodiment.
Fig. 6 is an explanatory view of an inserted state of the nostril plug of the first embodiment.
Fig. 7 is a back view of a nostril plug of a modification of the first embodiment.
Fig. 8 is a graph showing a test result of a monosyllable articulation test using the nostril plug of the first embodiment.
Fig. 9 is an explanatory view of an inserted state of a nostril plug of the modification.
Fig. 10 is a perspective view of a nostril plug of a second embodiment as viewed from an oblique front position.
Fig. 11 is a cross-sectional view of the nostril plug of the second embodiment.
Fig. 12 is a perspective view of a nostril plug of a modification of the second embodiment as viewed from an oblique front position.
Fig. 13 is a cross-sectional view of the nostril plug of the second embodiment.
Fig. 14 is a perspective view of a modification of a valve unit of the nostril plug of the second embodiment.
Fig. 15 is a cross-sectional view of the modification of the valve unit of the nostril plug of the second embodiment.
Fig. 16 is a cross-sectional view of the modification of the valve unit of the nostril plug of the second embodiment.
Fig. 17 is a cross-sectional view of a nostril plug of the modification of the second embodiment.
Fig. 18 is an explanatory view showing an inserted state of the nostril plug of the modification of the second embodiment.
Fig. 19 (not part of the invention) is a cross-sectional view of the nostril plug.
Fig. 20 (not part of the invention) is a cross-sectional view of the nostril plug.
Fig. 21 (not part of the invention) is a cross-sectional view of the nostril plug.
Fig. 22 is a perspective view of the nostril plug of the modification of the second embodiment.
Fig. 23 is a perspective view of a nostril plug of a third embodiment as viewed from an oblique front position.
Fig. 24 is an explanatory view of an inserted state of a support body of the nostril plug of the third embodiment.
Fig. 25 is a cross-sectional view of the nostril plug of a modification of the third embodiment.
Fig. 26 is an operational explanatory view of the nostril plug of the modification of the third embodiment.
Fig. 27 is an operational explanatory view of the nostril plug of the modification of the third embodiment.
Fig. 28 is a perspective view of the nostril plug of the modification as viewed from an oblique front position of the third embodiment.
Fig. 29 is an explanatory view showing the structure in the vicinity of a nose and a throat of a person.

Description of the Reference Numerals and Signs

A1:     nostril plug
11:     left housing
12:     right housing
13:     connecting bar
14:     valve unit
14a:    valve element
14b:    valve seat
14c:    ventilation opening
14d:    auxiliary bar
15:     scattering prevention wall

Best Mode for Carrying Out the Invention

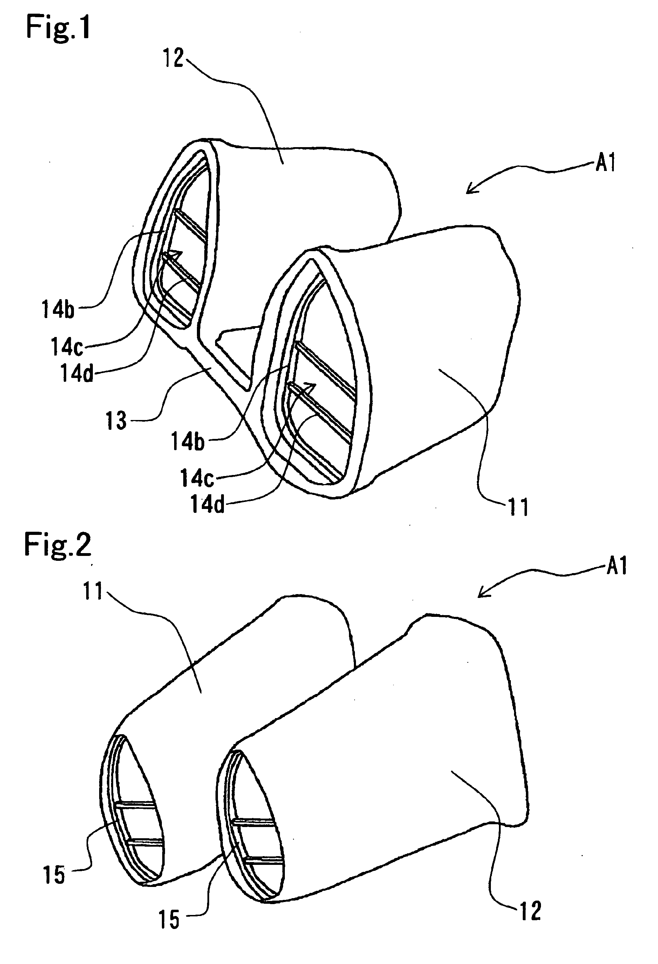

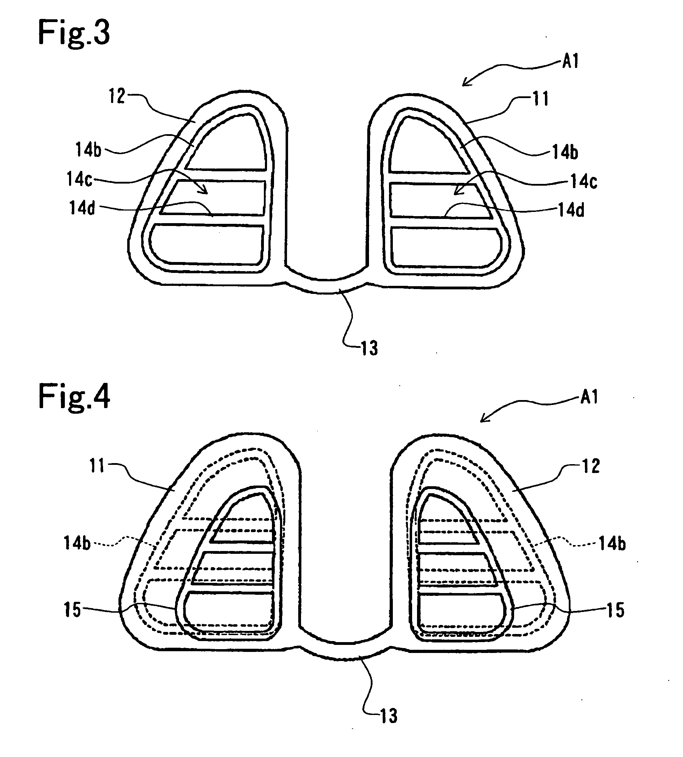

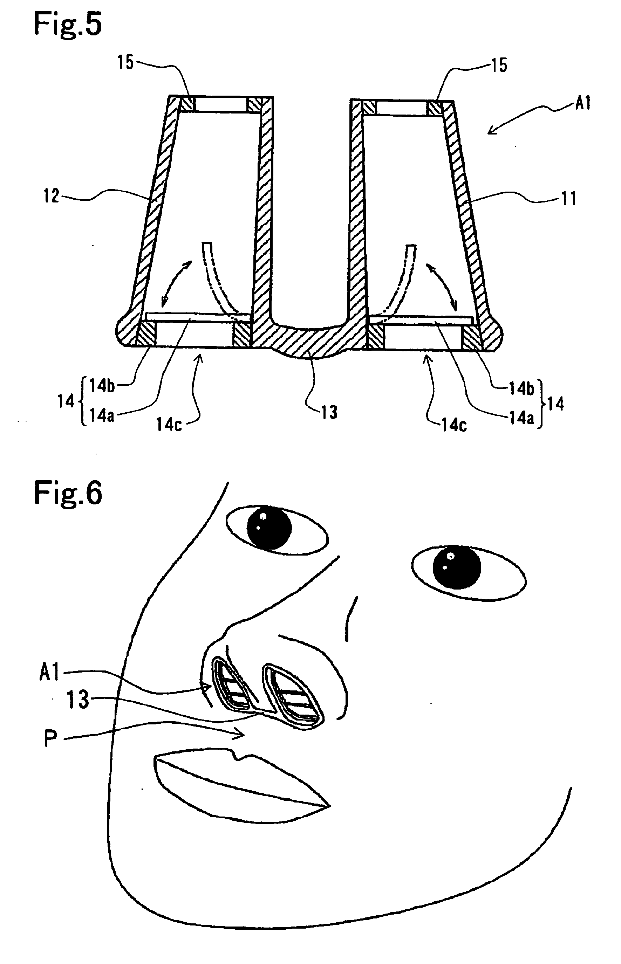

Fig. 1 is a perspective view of a nostril plug A1 of a first embodiment as viewed from an oblique front position. Fig. 2 is a perspective view of the nostril plug A1 of the first embodiment as viewed from an oblique rear position. Fig. 3 is a front view of the nostril plug A1 of the first embodiment. Fig. 4 is a backviewof the nostril plugA1 of the first embodiment. Fig. 5 is a cross-sectional view of the nostril plug A1 of the first embodiment.

The nostril plug A1 of the first embodiment includes a tubular left housing 11 inserted into a left nostril, a tubular right housing 12 inserted into a right nostril, a connecting bar 13 connecting a front end of the left housing 11 and a front end of the right housing 12, a valve unit 14 formed in the inside of the left housing 11 using the left housing 11 as a support body, and a valve unit 14 formed in the inside of the right housing 12 using the right housing 12 as a support body.

The left housing 11 and the right housing 12 are respectively formed in a tubular shape inscribed in the nostrils of a user having an articulatory disorder. The left housing 11 and the right housing 12 are formed by taking molds from the left and right nostrils of the user using the nostril plug A1 respectively and forming the left housing 11 and the right housing 12 in conformity with shapes of nostrils of the user using soft plastic. Here, the left housing 11 and the right housing 12 may be formed in plural kinds of representative shapes obtained by taking molds from nostrils of a plurality of persons, and the left housing 11 and the right housing 12 which conform to the user may be used.

Particularly, the left housing 11 and the right housing 12 are formed in a tapered shape which gradually decreases a diameter thereof toward a depth of each nostril thus allowing close fitting of the left housing 11 and the right housing 12 in the inside of the nostrils of the user.

The connecting bar 13 is made of hard plastic and connects the respective front ends of the left housing 11 and the right housing 12 while setting a distance between the left housing 11 and the right housing 12 smaller than a thickness of nasal septum of the user. Due to such a constitution, the left housing 11 and the right housing 12 clamps the nasal septum thus preventing the removal of the nostril plug A1 due to exhalation at the time of performing usual breathing.

Particularly, by arranging the connecting bar 13, as can be understood from an inserted state of the nostril plug A1 shown in Fig. 6, at a position where the connecting bar 13 is brought into contact with a philtrum P of the user during mounting, the connecting bar 13 can be concealed by a nose when the nostril plug A1 is inserted thus making the nostril plug A1 inconspicuous.

Further, when the connecting bar 13 is arranged at the position where the connecting bar 13 is brought into contact with the philtrum P, the nostril plug A1 can be easily removed by using the connecting bar 13 as a grip. That is, with respect to the nostril of a Japanese in general, a recessed space is often formed on a back-side portion of a nasal apex and hence, by fitting nasal-apex-side end portions of the left housing 11 and the right housing 12 in these recessed portions, it is possible to hold the left housing 11 and the right housing 12 in the inside of the nostrils in a stable manner. Further, in removing the nostril plug A1, by pinching the connecting bar 13 and pulling out the nostril plugA1, the left housing 11 and the right housing 12 can be rotated using the nasal-apex-side end portions of the left housing 11 and the right housing 12 fitted in the recessed portions of the nostrils as fulcrums and hence, the user can easily remove the nostril plug A1 without having feeling that the nostril plug A1 is caught by the nasal apex.

The valve units 14 formed in the inside of the left housing 11 and the right housing 12 are respectively, as shown in Fig. 5, constituted of a valve element 14a formed of an extremely thin rubber film or a plastic film which is resiliently deformable by warping, and a valve seat 14b having a ventilation opening 14c closed by the valve element 14a. In the nostril plug A1 of this embodiment, the valve seat 14b is formed in a nasal-apex-side opening portion of each of the left housing 11 and the right housing 12, and the valve element 14a is mounted on a side surface of the valve seat 14b facing the inside of each of the left housing 11 and the right housing 12.

Particularly, in this embodiment, the valve elements 14a are fixedly respectively mounted portions of the left housing 11 and the right housing 12 at positions close to the nasal septum. However, provided that the valve elements 14a can close the ventilation opening 14c formed in the valve seat 14b, the valve element 14a may be mounted at any positions.

As shown in Fig. 1 and Fig. 3, in this embodiment, the valve seats 14b are formed in a ring shape having the ventilation opening 14c of a round-cornered triangular shape which conforms to an opening shape of a distal end side of the left housing 11 and the right housing 12, wherein two parallel auxiliary bars 14d extend over each valve seat 14b such that the auxiliary bars 14d traverse the ventilation opening 14c thus preventing the valve element 14a from being removed to the outside from the ventilation opening 14c.

By mounting the valve element 14a on the side surfaces of the valve seats 14b which form the inside of the left housing 11 and the right housing 12, when the user inhales air inbreathing, as shown in Fig. 5, the valve elements 14a are deformed by warping and hence, the ventilation openings 14c are largely opened thus allowing the user to inhale air.

On the other hand, when the user performs exhalation, the valve element 14a closes the ventilation openings 14c and hence, leaking of exhalation from the nostrils can be suppressed whereby the articulatory disorder can be reduced. That is, the valve unit 14 constitutes a so-called one-directional valve.

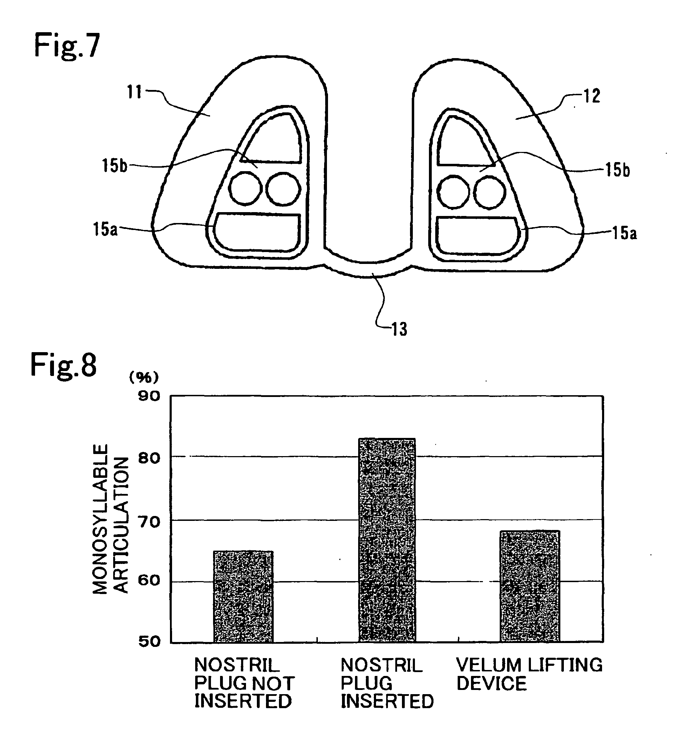

In Fig. 4 and Fig. 5, numeral 15 indicates scattering prevention walls which are mounted on rear-end side opening portion of the left housing 11 and the right housing 12 and prevent scattering of the broken valve elements 14a. When the valve element 14a is broken by any chance, the scattering prevention wall 15 receives such a valve element 14a. The scattering prevention walls 15 may be formed in any shape provided that the scattering prevention wall 15 has air permeability to an extent that the scattering prevention wall 15 does not impede breathing. Besides a shape shown in Fig. 4 which is similar to a shape of the valve seat 14b, as shown in Fig. 7, the scattering prevention wall 15 may be formed by extending a plate body 15b having circular ventilation openings over a frame 15a having a round-cornered triangular ring shape.

A result of a monosyllable articulation test using the nostril plug A1 having such a constitution is shown in Fig. 8. Here, the articulation means a degree of audibility of a sound (mainly spoken voice) and is used as one of criteria for determining whether or not a sound is properly uttered.

In the monosyllable articulation test, an examinee is asked to utter 100 pieces of monosyllables such as "a", "ga", "gyu" , and it is determined how accurately an examiner can listen to these monosyllables. A result of the monosyllable articulation test is determined based on a rate obtained by dividing the total number of syllables accurately listened with the total number of syllables in a chord chart as expressed by a following formula.

$$PA(\%) = (\text{total number of syllables accurately listened} /$$
$$\text{total number of syllables in chord chart}) \times 100$$

In general, the articulation is considered favorable when the rate is 85% or more and defective when the rate is 70% or less.

As shown in Fig. 8, although the articulation is approximately 65% in a state that the nostril plug A1 of this embodiment is not inserted, the articulation becomes approximately 83% when the nostril plug A1 is inserted and hence, there is no impression that the examiner has difficulty in listening. Further, when the velum lifting device is used in place of the nostril plug A1, the articulation is approximately 68% and no remarkable improvement of articulation is recognized.

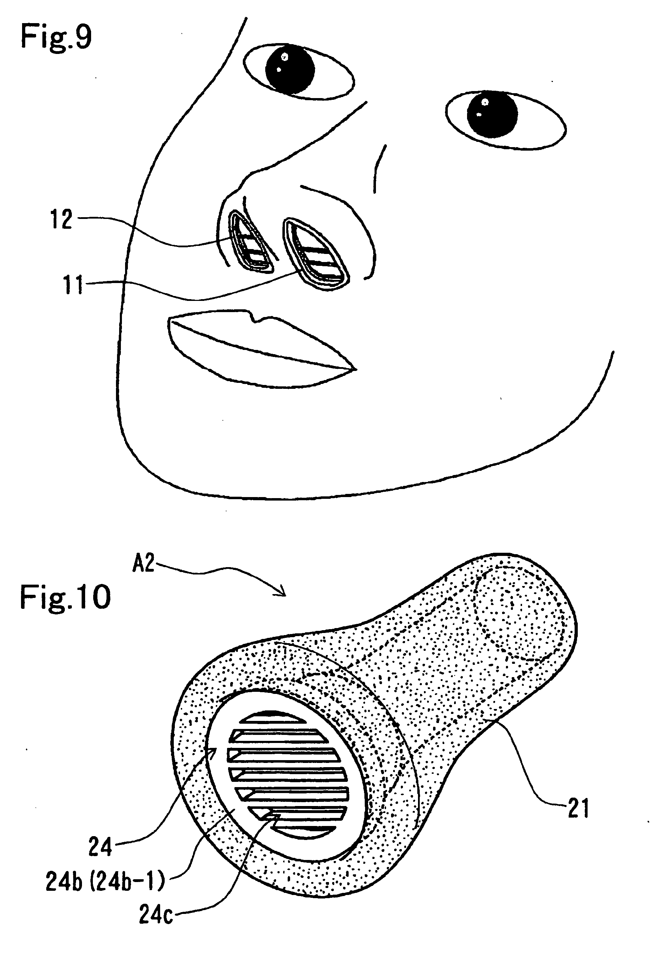

In the nostril plug A1 of the above-mentioned embodiment, the left housing 11 and the right housing 12 are connected to each other using the connecting bar 13. However, as shown in Fig. 9, the left housing 11 and the right housing 12 may be respectively inserted into the nostrils without using the connecting bar 13.

In this case, particularly, it is desirable to form the left housing 11 and the right housing 12 in conformity with shapes of nostrils of a user such that the left housing 11 and the right housing 12 are fitted in the nostrils as close as possible.

In the inserted state of the nostril plug A1 of this embodiment shown in Fig. 6 and Fig. 9, a distal end of the left housing 11 inserted into the left nostril and the distal end of the right housing 12 inserted into the right nostril are arranged at positions substantially equal to front end peripheries of the respective nostrils. However, by mounting the left housing 11 and the right housing 12 by positioning the distal end of the left housing 11 and the distal end of the right housing 12 inserted into the right nostril approximately several mm deeper than front end peripheries of the nostrils, it is possible to bring the nostril plug A1 into a state where a person who faces the user of the nostril plug A1 cannot observe the nostril plug A1 thus making the person who faces the user hardly recognize the presence of the nostril plug A1.

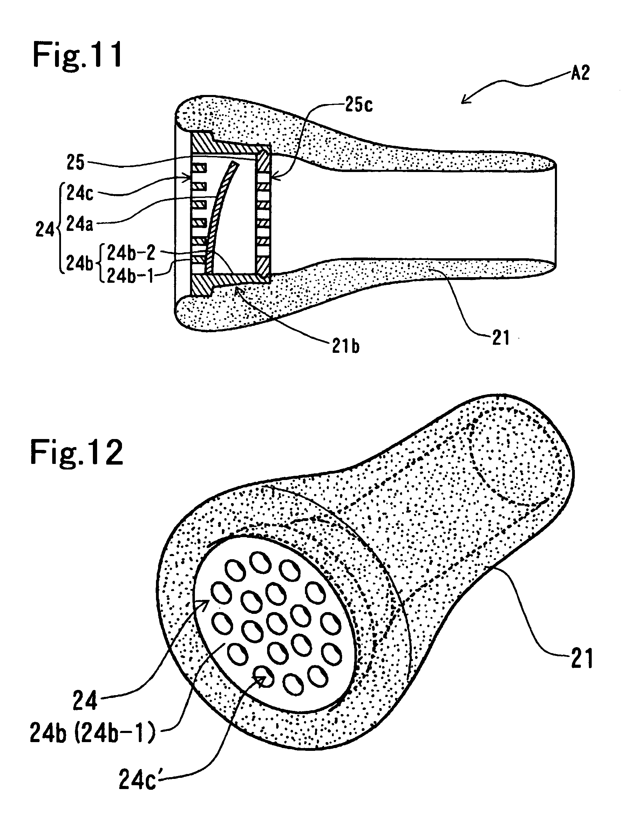

Next, a nostril plug of a second embodiment is explained. Fig. 10 is a perspective view of a nostril plug A2 of the second embodiment as viewed from an oblique front position. Fig. 11 is a cross-sectional view of the nostril plug A2 of the second embodiment.

The nostril plugA2 of the second embodiment also includes a tubular housing 21 inserted into a nostril, and a valve unit 24 which is arranged in the inside of the housing 21 constituting a support body.

In this embodiment, the housing 21 is formed of a tubular body made of soft plastic and is formed in a general shape which allows the easy insertion of the nostril. That is, an end portion of the housing 21 which forms a nasal apex side when the nostril plug A2 is inserted into the nostril has a diameter larger than a diameter of an end portion of the housing 21 which forms a depth side of the nostril. Here, the housing 21 may be formed by taking a mold from a left nostril or a right nostril of a user suffering from the articulatory disorder.

The valve unit 24 is constituted of a valve element 24a formed of an extremely thin rubber film or a plastic film which is resiliently deformable by warping, and a valve seat 24b having a ventilation opening 24c closed by the valve element 24a.

Particularly, in this embodiment, the valve seat 24b is constituted of a circular plate body 24b-1 in which the ventilation opening 24c is formed and a cylindrical tubular wall 24b-2 formed on an outer periphery of theplatebody24b-1, wherein the plate body 24b-1 is formed on one end periphery of the tubular wall 24b-2. A plurality of ventilation openings 24c having a slit shape are formed in the plate body 24b-1 in parallel to each other. Here, the shape of the ventilation opening 24c is not limited to a slit shape and may be formed in a proper shape including a ventilation opening 24c' having a circular shape shown in Fig. 12, for example.

The valve element 24a is mounted on a side surface of the plate body 24b-1 facing the inside of the tubular wall 24b-2 so as to close the ventilation opening 24c.

Further, a scattering prevention wall 25 for preventing scattering of the broken valve element 24a is mounted on an end portion of the tubular wall 24b-2 facing the plate body 24b-1. When the valve element 24a is broken by any chance, the valve element 24a is received by the scattering prevention wall 25. In this embodiment, the scattering prevention wall 25 is formed of a circular plate body which is mountable on the tubular wall 24b-2, and forms a plurality of slit-shaped ventilation openings 25c therein for preventing the scattering prevention wall 25 from constituting an obstacle of breathing.

By inserting the nostril plugA2 having such a constitution into a nostril, when the user inhales air in breathing, as shown in Fig. 11, the valve elements 24a are deformed by warping and hence, the ventilation openings 24c are largely opened thus allowing the user to inhale air. On the other hand, when the user performs exhalation, the valve element 24a closes the ventilation openings 24c and hence, leaking of exhalation from the nostrils can be suppressed whereby the articulatory disorder can be reduced.

Particularly, in the nostril plug A2 of this embodiment, a mounting recessed portion 21b formed in a recessed shape to be fitted on the tubular wall 24b-2 of the valve seat 24b is formed in an inner peripheral surface of the tubular housing 21 on a nasal apex side, and the valve unit 24 is removably mounted in the mounting recessed portion 21b. The mounting recessed portion 21b is formed in an expanded manner toward the nasal apex side to facilitate fitting of the valve unit 24 into the mounting recessed portion 21b.

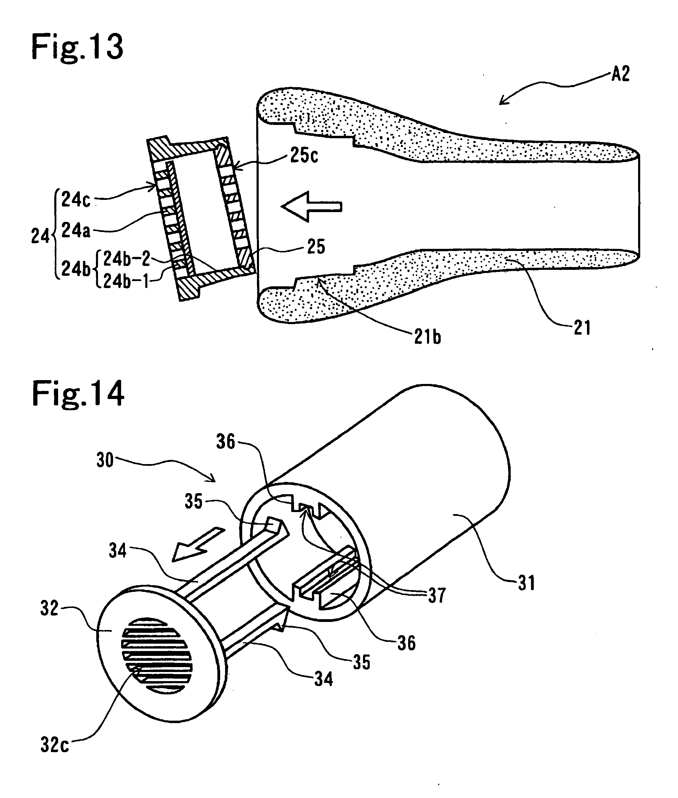

The valve unit 24 is fitted in the mounting recessed portion 21b to an extent that, when the valve unit 24 is fitted in the mounting recessed portion 21b, the valve unit 24 is not removed due to usual breathing, while when a large quantity of air flows in a nasal cavity side at the time of sneezing or the like and the pressure in the inside of the nostril is elevated to a predetermined level or more, the valve unit 24 is removed from the mounting recessed portion 21b due to the pressure as shown in Fig. 13.

The pressure at which the valve unit 24 is removed from the housing 21 is adjusted by a fitting strength between the valve unit 24 and the mounting recessed portion 21b. To be more specific, the fitting strength is adjusted by setting a diameter size of the tubular wall 24b-2 of the valve unit 24 slightly larger than a diameter size of the corresponding mounting recessed portion 21b. Alternatively, the fitting strength between the valve unit 24 and the mounting recessed portion 21b can be also adjusted by adjusting a size of a contact area between the tubular wall 24b-2 of the valve unit 24 and the mounting recessedportion 21b. For example, the fitting strength between the valve unit 24 and the mounting recessed portion 21b may also be adjusted by forming a non-contact surface on an outer peripheral surface of the tubular wall 24b-2 where the tubular wall 24b-2 is not brought into contact with the mounting recessed portion 21b by cutting a portion of the outer peripheral surface of the tubular wall 24b-2.

In this manner, according to the nostril plug A2 of this embodiment, by removablymounting the valve unit 24 in the housing 21, by forming an outer peripheral shape of the housing 21 into a proper shape which conforms to a shape of a nostril of a user and, at the same time, by forming an inner peripheral shape of the housing 21 into a standardized predetermined shape, the valve unit 24 can be used in common and hence, a manufacturing cost can be reduced. Further, depending on the degradation of the housing 21 and the degradation of the valve unit 24, only the housing 21 or only the valve unit 24 canbe exchanged thus realizing the reduction of a maintenance cost.

Further, when a pressure inside the nostril is sharply increased due to sneezing or the like, the valve unit 24 is removed from the housing 21 due to the pressure for alleviating the pressure inside the nostril thus preventing the removal of the housing 21 from the nostril. Accordingly, the valve unit 24 can be easily mounted again after the sneeze.

When the pressure in the inside of the nostril is sharply increased due to sneezing or the like, instead of alleviating the pressure by removing the valve unit 24 from the housing 21, a ventilation means for alleviating the pressure may be mounted on the valve unit per se.

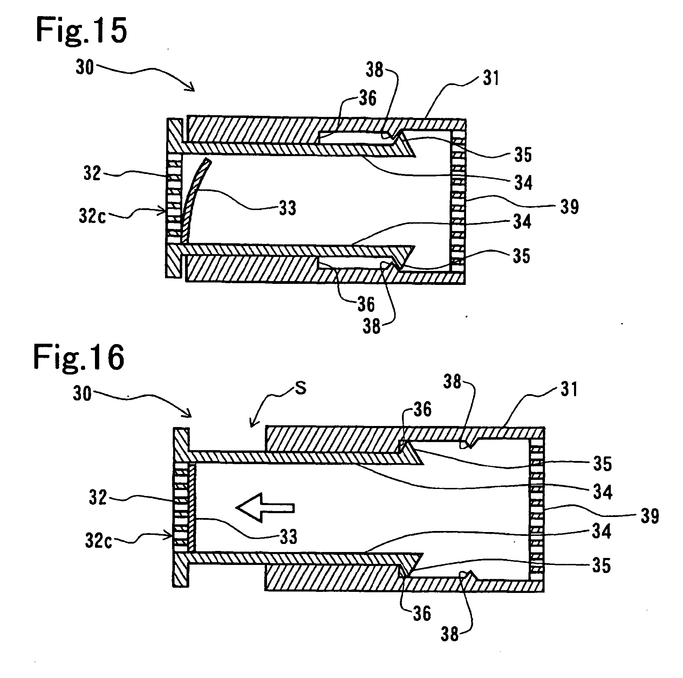

To be more specific, as shown in Fig. 14 and Fig. 15, the valve unit 30 includes a tubular frame 31 having both ends thereof opened, a planar valve seat 32 which is mounted on one opening end portion of the frame 31 and is configured to close the opening end, and a valve element 33 which is mounted on a side surface of the valve seat 32 facing the inside of the frame 31 and is configured to close a ventilation opening 32c formed in the valve seat 32, on a surface side of the valve seat 32 on which the valve element 33 is arranged, a guide arm 34 brought into contact with an inner peripheral surface of the frame 31 is arranged in a projecting manner, and the valve seat 32 is configured to be advanced or retracted in the longitudinal direction of the frame 31.

Particularly, on a distal end of the guide arm 34, a hook 35 which is engaged with a bulging projecting portion 36 formed on an inner peripheral surface of the frame 31 is formed. By engaging the hook 35 with the projecting portion 36, the removal of the valve seat 32 from the frame 31 can be prevented.

Further, the guide groove 37 in which the guide arm 34 is fitted is formed in the projecting portion 36 and hence, the guide arm 34 is extendible and retractable in the longitudinal direction of the frame 31 while being restricted by the guide groove 37.

Further, on an inner peripheral surface of the frame 31, as shown in Fig. 15, an engaging member 38 which is engaged with the hook 35 of the guide arm 34, in a state that the valve seat 32 is most retracted, is formed. Due to the engagement of the hook 35 with the engaging member 38, the guide arm 34 prevents the valve seat 32 from advancing due to a pressure of exhalation in usual breathing. On the other hand, when a pressure in the inside of the nostril reaches a predetermined value or more due to sneezing or the like, the valve seat 32 is pushed by the pressure so that an engagement state between the hook 35 and the engaging member 38 is released and hence, the valve seat 32 is advanced. Accordingly, as shown in Fig. 16, a gap S is formed between the valve seat 32 and the frame 31 thus releasing the pressure from the gap S. In Fig. 15 and Fig. 16, numeral 39 indicates a scattering prevention wall forpreventingscatteringof the valve element 33 formed on the frame 31.

Due to the engagement of the hook 35 of the guide arm 34 with the projecting portion 36, there is no possibility that the valve seat 32 is removed from the frame 31. When the gap S is generated between the valve seat 32 and the frame 31, the valve seat 32 is pushed to the frame 31 side thus engaging the hook 35 of the guide arm 34 with the engaging member 38 of the frame 31 thus returning the valve seat 32 to a state where the articulatory disorder can be reduced.

Accordingly, the pressure inside the nostril can be alleviated without removing the valve unit 30 from the housing 21 and hence, missing of the valve unit 30 removed from the housing 21 can be prevented whereby a user can sneeze without embarrassment in sneezing.

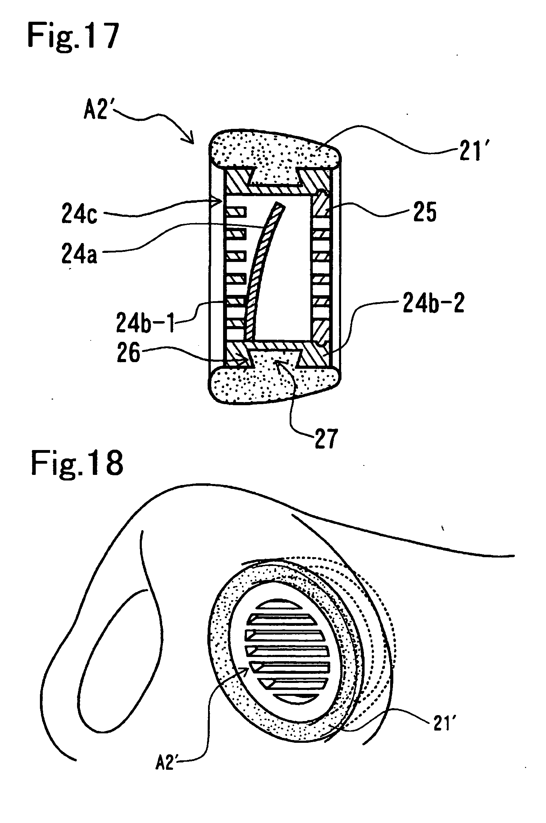

The housing 21 may not be always made of the above-mentioned soft plastic, and may be formed using low-repulsive resilient urethane foam. Fig. 17 is a cross-sectional view of a nostril plug A2' using a housing 21' made of low-repulsive resilient urethane foam. Since the low-repulsive resilient urethane foam can be depressed into a small piece using fingertips and can gradually restore an original shape when depressing is stopped and hence, it is possible to bring the urethane foam into close contact with the nostril whereby the housing 21' can be shortened compared to the housing 21 made of soft plastic.

In the nostril plug A2' using the housing 21' made of low-repulsive resilient urethane foam, the housing 21' per se is easily deformed. Accordingly, to firmly hold a valve unit 24 by the housing 21', amortise 26 is formed on an outer peripheral surface of a tubular wall 24b-2 in the valve unit 24 along the circumferential direction, a dowel 27 which is fitted in the mortise 26 is formed in an inner peripheral surface of the housing 21', and the valve unit 24 is firmly mounted in the housing 21' due to the fitting engagement of the dowel 27 and the mortise 26.

The nostril plug A2' using the housing 21' made of low-repulsive resilient urethane foam can be inserted into the nostril as shown in Fig. 18 by depressing the housing 21' portion with fingers before inserting the nostril plug A2' into the nostril and by inserting the nostril plug A2' into the nostril before the housing 21' is restored to an original shape. The nostril plug A2' conforms to a shape of the nostril which differs for every individual by properly deforming the housing 21' and hence, it is unnecessary to take a mold of the nostril. Further, there is no difference in use of the nostril plug A2' between the left nostril and the right nostril and hence, it is sufficient to preliminarily prepare plural kinds of housings 21' which differ in size of outer peripheral diameter corresponding to sizes of the nostrils whereby it is possible to further reduce a manufacturing cost.

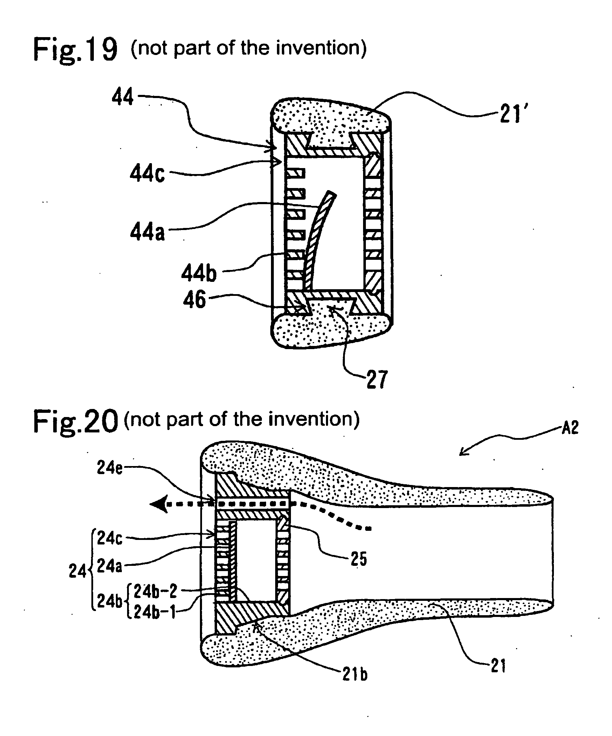

In the above-mentioned embodiment, the valve element 24a is mounted such that the valve element 24a of the valve unit 24 is configured to substantially completely close the ventilation opening 24c formed in the valve seat 24b. However, it is not always necessary for the valve element 24a to substantially completely close the ventilation opening 24c. For example, as shown in Fig. 19 (not part of the invention), a valve unit 44 may be configured such that a plurality of ventilation openings 44c are formed in a valve seat 44b, and a size or a shape of a valve element 44a is set to close some ventilation openings 44c with the valve element 44a thus forming the ventilation openings 44c always in an opened state.

In this manner, by forming the ventilation openings 44c always in an opened state, it is possible to leak a portion of exhalation from the ventilation openings 44c in an opened state thus improving a tone of "m" or "n" which constitutes nasal consonant.

Here, instead of dividing the plurality of ventilation openings 44c into the ventilation openings 44c which are closed by the valve element 24a and the ventilation openings 44c which are not closed by the valve element 24a, one ventilation opening 44c may be partially closed by the valve element 24a thus forming a region closed by the valve element 24a and a region which is not closed by the valve element 24a. In Fig. 19 (not part of the invention), numeral 46 indicates a mortise in which a dowel 27 of a housing 21' made of low-repulsive resilient urethane foam is fitted.

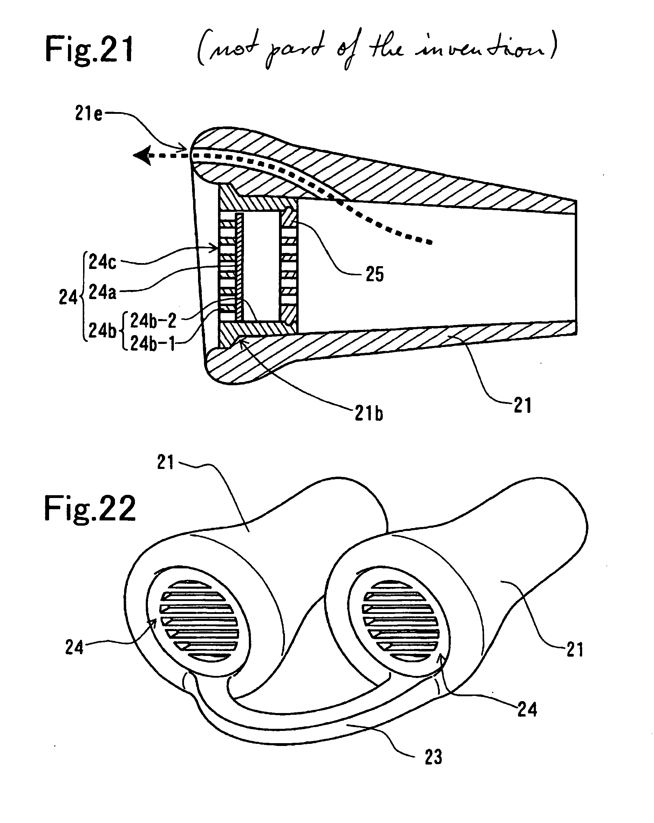

Alternatively, as shown in Fig. 20 (not part of the invention), with respect to the nostril plug A2 shown in Fig. 11, a ventilation passage 24e which allows a predetermined flow rate of air to pass therethrough may be formed in the valve seat 24b thus leaking a portion of the exhalation or, as shown in Fig. 21, a ventilation passage 21e which allows a predetermined flow rate of air to pass therethrough may be formed in the housing 21 thus leaking a portion of the exhalation. Also in such cases, it is possible to leak a portion of the exhalation from the ventilation passages 21e, 24e thus improving a tone of "m" or "n" which constitutes a nasal consonant.

The above-mentioned nostril plug A2 of the second embodiment may be individually inserted into the left nostril or the right nostril or, as shown in Fig. 22, the housing 21 of the first nostril plug A2 inserted into the left nostril and the housing 21 of the second nostril plug A2 inserted into the right nostril may be integrally formed by connecting these housings 21 using a connecting bar 23.

Particularly, the connecting bar 23 performs not only the function of connecting a distal end of the housing 21 of the first nostril plugs A2 and a distal end of the housing 21 of the second nostril plugs A2 but also a function of making the connecting bar 23 inconspicuous in the same manner as the nostril plugs A1 of the first embodiment by arranging the connecting bar 23 at a position where the connecting bar 23 is brought into contact with a philtrum at the time of inserting the first nostril plugs A2 into the left nostril and the second nostril plugs A2 into the right nostril.

Further, using the connecting bar 23 as a grip, handling of the nostril plugA2 canbe enhanced. Particularly, the nostril plug A2 can be extremely easily removed from the nostril using the connecting bar 23 as the grip.

Further, in connecting the housing 21 of the first nostril plug A2 inserted into the left nostril and the housing 21 of the second nostril plug A2 inserted into the right nostril using the connecting bar 23, out of the valve unit 24 of the first nostril plug A2 and the valve unit 24 of the second nostril plug A2, one valve unit may be configured to be more easily removed from the housing 21 than the other valve unit 24 and hence, when the valve unit 24 is removed by sneezing or the like, only one valve unit 24 is removed thus facilitating re-mounting of the valve unit 24.

In general, the smoothness of air flow often differs between the left nostril and the right nostril of a person. Accordingly, it is desirable to adjust the easiness of removal of the valve units 24 by selecting either one of left and right nostril plugs A2 in response to a state of a user who uses the nostril plugs A2 connected by the connecting bar 23. The adjustment of easiness of removal of the valve units 24 can be easily performed by adjusting a contact area between a mounting recessed portion 21b of the housing 21 and the valve unit 24.

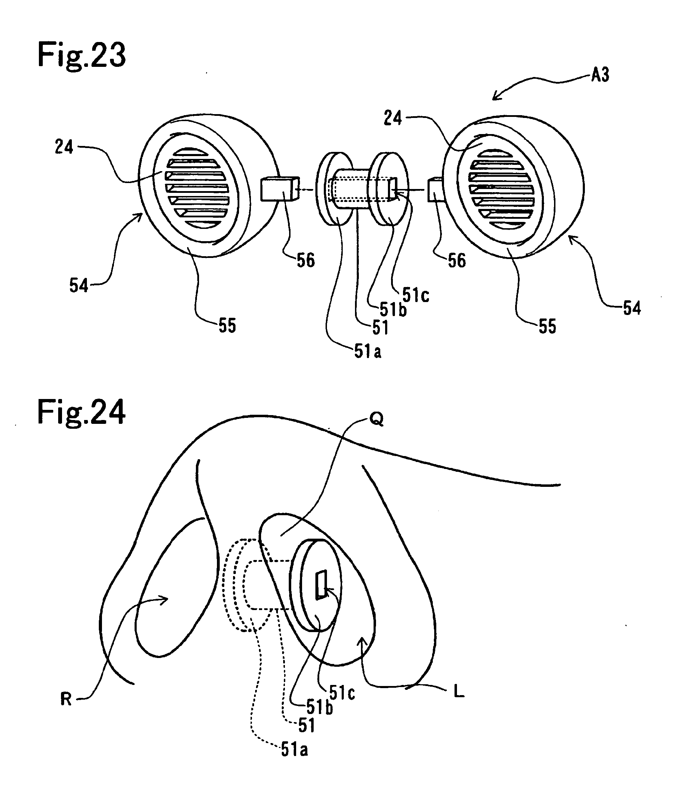

Next, a nostril plug of a third embodiment is explained. Fig. 23 is a perspective view of a nostril plug A3 of the third embodiment as viewed from an oblique front position.

The nostril plug A3 of the third embodiment is also constituted of a valve unit 54 and a support body 51 on which the valve unit 54 is mounted. Particularly, as shown in Fig. 24, the support body 51 is formed of a rod-shaped body which has one end portion thereof arranged in the inside of a left nostril L after being made to pass through a through hole formed in a nasal septum Q and the other end portion thereof arranged in the inside of a right nostril R after being made to pass through the through hole formed in the nasal septum Q.

A first flange 51a and a second flange 51b are respectively formed on both ends of the support body 51, and the support body 51 is prevented from being removed through the through holes formed in the nasal septum Q due to the first flange 51a and the second flange 51b. Here, in this embodiment, the first flange 51a is integrally formed with the support body 51 and, at the same time, the second flange 51b can be threadedly mounted on the support body 51. In mounting the support body 51 in a nasal septum Q, the support body 51 is inserted into the through hole formed in the nasal septum Q and, thereafter, the second flange 51b is threadedly mounted on an end portion of the support body 51.

The valve unit 54 is constituted of the above-mentioned valve unit 24 of the above-mentioned second embodiment and a tubular connecting jig 55 in which the valve unit 24 is fitted. The valve unit 24 is removably mounted in the connecting jig 55. To obviate the duplication of explanation, the explanation of the valve unit 24 of the second embodiment is omitted.

The connecting jig 55 is made of soft plastic and is formed in a tubular shape inscribed in a left nostril L and a right nostril R of a user having an articulatory disorder. On an outer peripheral surface of the connecting jig 55, a rod 56 which is inserted into a fitting hole 51c formed in an end portion of the support body 51 is mounted.

By inserting the valve unit 54 into the left nostril L or the right nostril R while inserting the rod 56 into the fitting hole 51c of the support body 51 mounted in the nasal septum Q, the nostril plug A3 is formed.

By constituting the nostril plug A3 using the support body 51 mounted in the nasal septum Q and the valve unit 54 mounted on the support body in this manner, this embodiment can provide the relatively light-weighted nostril plug A3.

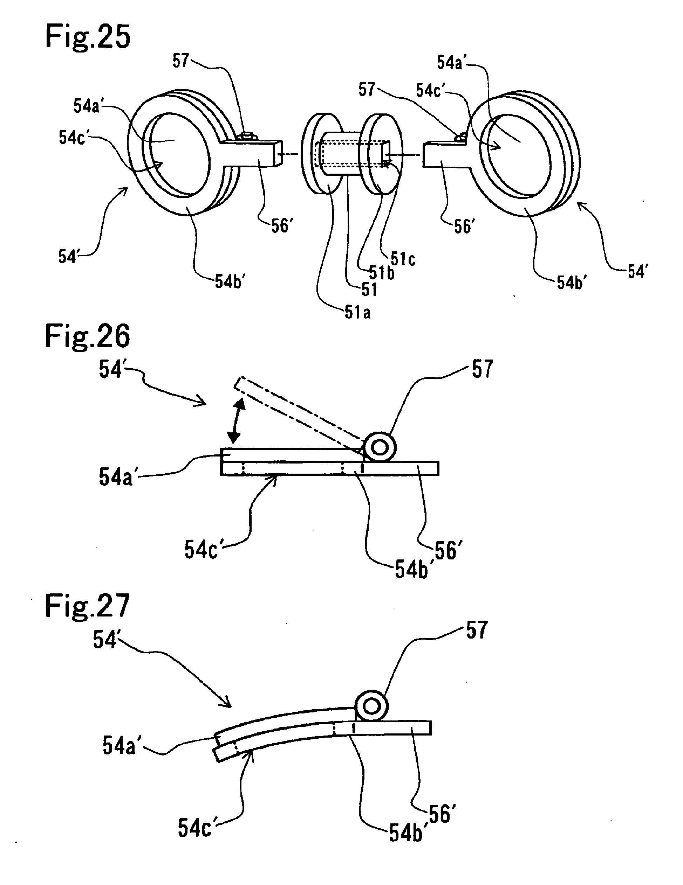

As shown in Fig. 25 and Fig. 26, a valve unit 54' may be constituted of a ring-shaped valve seat 54b' having a rod 56' to be inserted into a fitting hole 51c of a support body 51, and a valve element 54a' mounted on a valve seat 54b' for closing a ventilation opening 54c'.

The valve seat 54b' is formed of a planar body having resiliency such as a plastic plate, and the rod 56' is mounted on an outer periphery of the ring- shaped valve seat 54b' in place. Here, the valve seat 54b' may be formed of a planar plate made of shape memory alloy. When the valve seat 54b' is made of shape memory alloy, in removing the valve unit 54' from the support body 51, the valve unit 54' can be easily removed by properly deflecting and removing the valve seat 54b'. Further, after removing the valve unit 54', the valve seat 54b' is immersed in hot water of a predetermined temperature so that the valve seat 54b' can easily restore an original shape and can be used again.

The valve element 54a' is configured to overlap the ring-shaped valve seat 54b' to close the ventilation opening 54c'. Particularly, by connecting one end of the valve element 54a' to a hinge 57 mounted on the rod 56', the valve element 54a' is rotatable by the hinge 57. In this embodiment, the valve element 54a' is rotatable using the hinge 57 and hence, the valve element 54a' is not always required to possess resiliency. In this embodiment, the valve element 54a' is formed of a plastic plate in the same manner as the valve seat 54b'. Here, the valve element 54a' may be formed of a rubber film or a plastic film which is resiliently deformable by warping.

As shown in Fig. 26, the valve unit 54' having such a constitution can, at the time of inhaling air inbreathing, inhale air by bringing the ventilation opening 54c' into an opened state with the rotation of the valve element 54a' and, at the same time, in inhaling air, the valve element 54a' close the ventilation opening 54c' thus preventing leaking of exhalation from the left nostril L and the right nostril R. Accordingly, the nostril plug can reduce the articulatory disorder.

Further, when a pressure inside the nostril reaches a predetermined pressure or more due to sneezing or the like, as shown in Fig. 27, the valve seat 54b' and the valve element 54a' are resiliently deformed due to such a pressure and are deflected toward a nasal apex side and hence, a gap can be formed between the valve seat 54b' and the left nostril L or the right nostril R whereby the pressure can be rapidly released through the gap.

As another embodiment, in place of using the ring-shaped valve seat 54b' , for example, as shown in Fig. 28, a valve element 54a" may be supported in a closed state using an arm-shaped valve seat 54b". Particularly, by forming the valve seat 54b" into a bifurcated arm shape, the valve element 54a" can be supported in a stable manner and, at the same time, when a pressure in the inside of a nostril reaches a predetermined pressure or more by sneezing or the like, the valve seat 54b" canbeeasilydeflected to facilitate releasing of the pressure.

Industrial Applicability

With a provision of the nostril plug having the valve unit which functions as one-directional valve capable of suppressing passing of exhalation while allowing passing of inhaled air in breathing, the articulatory disorder of a patient having functional articulatory disorder can be reduced while reducing a physical burden imposed on the patient as much as possible.

**Claims**

1. A nostril plug (A1, A2, A3) for suppressing leakage of exhalation from a nose by insertion of the nostril plug (A1, A2, A3) into a nostril, the nostril plug (A1, A2, A3) comprising:

   a valve unit (19, 24, 30, 54, 54') for suppressing only passage of exhalation in breathing, the valve unit comprising a valve element (14a, 24a, 33, 54a'), and a valve seat (14b, 24b, 32, 54b') having a ventilation opening (14c, 24c, 54c') wherein the valve element is constructed to close the ventilation opening formed in the valve seat by exhalation; and
   a support body (11, 12) for supporting the valve unit (14, 24, 30, 54, 54'), the support body mounting the valve unit thereon and configured to be arranged in the inside of the nostril (A1, A2, A3).

2. A nostril plug (A1, A2, A3) according to claim 1, wherein the support body is formed of a tubular body inscribed in the nostril, and the valve unit is removably fitted in the support body.

3. A nostril plug (A1, A2, A3) according to claim 2, wherein the valve unit is configured to be separated from the support body by pressure when the pressure inside the nostril reaches a predetermined level or more by exhalation.

4. A nostril plug (A1, A2, A3) according to claim 3, wherein the nostril plug includes a first support body to be inserted into a left nostril, a second support body to be inserted into a right nostril, and a connecting bar which connects the first support body and the second support body, and the connecting bar is arranged at a position where the connecting bar is brought into contact with a philtrum when the first support body is inserted into the left nostril and the second support body is inserted into the right nostril.

5. A nostril plug (A1, A2, A3) according to claim 3, wherein the nostril plug includes a first support body to be inserted into a left nostril, a second support body to be inserted into a right nostril, and a connecting bar which connects the first support body and the second support body, and
   the valve unit mounted on either one of the support bodies is configured to be more easily separated from the support body than the other valve unit.

6. A nostril plug(A1, A2, A3) according to any one of claims 1 to 5, wherein the valve unit includes a valve seat formed with a plurality of ventilation openings and a valve element which closes the ventilation openings.

7. A nostril plug (A1, A2, A3) according to claim 1, wherein the valve unit includes a tubular frame having both ends thereof opened, a planar valve seat which is mounted on one opening end side of the frame and is configured to close the opening end, and a valve element which is mounted on a side surface of the valve seat facing the inside of the frame and is configured to close a ventilation opening formed in the valve seat,
   on a surface side of the valve seat on which the valve element is arranged, a guide arm having a hook which is engaged with a projecting portion formed on an inner peripheral surface of the frame is arranged along the longitudinal direction of the frame, and the valve seat is configured to be advanced or retracted in the longitudinal direction of the frame by restricting the guide arm with the frame, and

in a state that a pressure inside the nostril reaches a predetermined level or more through exhalation, the valve seat is advanced from the frame due to the pressure to form a gap thus releasing the pressure.

8. A nostril plug (A1, A2, A3) according to claim 1, wherein the support body is formed of a rod-shaped body which is configured to be inserted into a through hole formed in a nasal septum, and has one end portion thereof arranged in the inside of the left nostril and the other end portion thereof arranged in the inside of the right nostril, and
the valve unit includes a valve seat formed of a resilient body mounted on an end portion of the support body and a valve element which is configured to close a ventilation opening formed in the valve seat, and
in a state that a pressure inside the nostril reaches a predetermined level or more through exhalation, the valve seat is configured to be resiliently deformed due to the pressure to form a gap between the valve seat and the nostril thus releasing the pressure.

**Patentansprüche**

1. Nasenlochstopfen (A1, A2, A3) zum Unterdrücken einer Ausatmungsleckage einer Nase durch Einführen des Nasenlochstopfens (A1, A2, A3) in ein Nasenloch, der Nasenlochstopfen (A1, A2, A3) mit:

   einer Ventileinheit (14, 24, 30, 54, 54'), um nur die Ausatmungspassage beim Atmen zu unterdrücken, wobei die Ventileinheit ein Ventilelement (14a, 24a, 33, 54a') und einen Ventilsitz (14b, 24b, 32, 54b') mit einer Ventilationsöffnung (14c, 24c, 54c') aufweist, wobei das Ventilelement gestaltet ist, um die in dem Ventilsitz ausgebildete Ventilationsöffnung durch Ausatmen zu schließen; und
   einem Stützkörper (11, 12) zum Unterstützen der Ventileinheit (14, 24, 30, 54, 54'), wobei der Stützkörper auf sich die Ventileinheit montiert hat und eingerichtet ist, in dem Inneren des Nasenlochs (A1, A2, A3) angeordnet zu sein.

2. Nasenlochstopfen (A1, A2, A3) nach Anspruch 1, bei dem der Stützkörper aus einem in dem Nasenloch umschriebenen röhrenförmigen Körper ausgebildet ist, und die Ventileinheit entfernbar in den Stützkörper eingepasst ist.

3. Nasenlochstopfen (A1, A2, A3) nach Anspruch 2, bei dem die Ventileinheit eingerichtet ist, von dem Stützkörper durch Druck getrennt zu werden, wenn der Druck in dem Nasenloch einen festgelegten Pegel oder mehr durch Ausatmen erreicht.

4. Nasenlochstopfen (A1, A2, A3) nach Anspruch 3, wobei der Nasenlochstopfen einen ersten Stützkörper, der in ein linkes Nasenloch einzuführen ist, einen zweiten Stützkörper, der in ein rechtes Nasenloch einzuführen ist, und einen Verbindungsstab aufweist, der den ersten Stützkörper und den zweiten Stützkörper verbindet, und der Verbindungsstab in einer Position angeordnet ist, wo der Verbindungsstab mit einem Philtrum in Kontakt gebracht wird, wenn der erste Stützkörper in das linke Nasenloch eingeführt wird und der zweite Stützkörper in das rechte Nasenloch eingeführt wird.

5. Nasenlochstopfen (A1, A2, A3) nach Anspruch 3, wobei der Nasenlochstopfen einen ersten Stützkörper, der in ein linkes Nasenloch einzuführen ist, einen zweiten Stützkörper, der in ein rechtes Nasenloch einzuführen ist, und einen Verbindungsstab aufweist, der den ersten Stützkörper und den zweiten Stützkörper verbindet, und die auf einem der Stützkörper montierte Ventileinheit eingerichtet ist, einfacher von dem Stützkörper getrennt zu werden als die andere Ventileinheit.

6. Nasenlochstopfen (A1, A2, A3) nach einem der Ansprüche 1 bis 5, bei dem die Ventileinheit einen Ventilsitz, der mit einer Vielzahl von Ventilationsöffnungen ausgebildet ist, und ein Ventilelement, das die Ventilationsöffnungen schließt, aufweist.

7. Nasenlochstopfen (A1, A2, A3) nach Anspruch 1, wobei die Ventileinheit einen röhrenförmigen Rahmen aufweist, bei dem beide Enden geöffnet sind, einen planen Ventilsitz, der auf einer der Öffnungsseiten des Rahmens montiert ist und eingerichtet ist, das Öffnungsende zu schließen, und ein Ventilelement, das auf einer Seitenfläche des Ventilsitzes montiert ist, die der Innenseite des Rahmens gegenübersteht und eingerichtet ist, eine in dem Ventilsitz ausgebildete Ventilationsöffnung zu schließen,
auf einer Flächenseite des Ventilsitzes, auf dem das Ventilelement angeordnet ist, ist ein Führungsarm mit einem Haken, der mit einem Vorsprungabschnitt in Eingriff ist, der auf einer inneren Umfangsfläche des Rahmens ausgebildet ist, entlang der Längsrichtung des Rahmens angeordnet, und der Ventilsitz ist eingerichtet, in der Längsrichtung des Rahmens durch Einschränken des Führungsarms mit dem Rahmen vor- oder zurückgeschoben zu werden, und in einem Zustand, in dem ein Druck durch Ausatmen innerhalb des Nasenlochs einen festgelegten Pegel oder mehr erreicht, der Ventilsitz von dem Rahmen aufgrund des Drucks vorgeschoben wird, um einen Spalt auszubilden und somit den Druck entweichen zu lassen.

8. Nasenlochstopfen (A1, A2, A3) nach Anspruch 1, bei dem der Stützkörper aus einem stabförmigen Körper ausgebildet ist, der eingerichtet ist, in ein in einem nasalen Septum ausgebildetem Durchgangsloch eingeführt zu werden, und einer seiner Endabschnitte im Inneren des linken Nasenlochs angeordnet ist und sein anderer Endabschnitt im Inneren des rechten Nasenlochs angeordnet ist, und

die Ventileinheit einen Ventilsitz aufweist, der aus einem elastischen Körper ausgebildet ist, der auf einem Endabschnitt des Stützkörpers montiert ist, und ein Ventilelement, das eingerichtet ist, eine in dem Ventilsitz ausgebildete Ventilationsöffnung zu schließen, und

in einem Zustand, in dem ein Druck innerhalb des Nasenlochs einen festgelegten Pegel oder mehr durch Ausatmen erreicht, der Ventilsitz eingerichtet ist, aufgrund des Drucks elastisch verformt zu werden, um einen Spalt zwischen dem Ventilsitz und dem Nasenloch auszubilden und somit den Druck entweichen zu lassen.


## Revendications

1. Bouche-narine (A1, A2, A3) pour supprimer une fuite d'expiration par le nez par insertion du bouche-narine (A1, A2, A3) dans une narine, le bouche-narine (A1, A2, A3) comprenant :

une unité formant soupape (14, 24, 30, 54, 54') pour supprimer seulement le passage d'expiration lors de la respiration, l'unité formant soupape comprenant un élément de soupape (14a, 24a, 33, 54a'), et un siège de soupape (14b, 24b, 32, 54b') ayant une ouverture de ventilation (14c, 24c, 54c') dans lequel l'élément de soupape est construit pour fermer l'ouverture de ventilation formée dans le siège de soupape par l'expiration ; et un corps support (11, 12) pour supporter l'unité formant soupape (14, 24, 30, 54, 54'), le corps support montant l'unité formant soupape sur ce dernier et agencé pour être disposé à l'intérieur de la narine (A1, A2, A3).

2. Bouche-narine (A1, A2, A3) selon la revendication 1, dans lequel le corps support est formé d'un corps tubulaire inscrit dans la narine, et l'unité formant soupape est ajustée de façon amovible dans le corps support.

3. Bouche-narine (A1, A2, A3) selon la revendication 2, dans lequel l'unité formant soupape est configurée pour être séparée du corps support par pression lorsque la pression à l'intérieur de la narine atteint un niveau prédéterminé ou plus par expiration.

4. Bouche-narine (A1, A2, A3) selon la revendication 3, dans lequel le bouche-narine inclut un premier corps support à insérer dans la narine gauche, un second corps support à insérer dans la narine droite, et une barre de liaison qui relie le premier corps support et le second corps support, et

la barre de liaison est agencée à une position où la barre de liaison est mise en contact avec un sillon sous-nasal lorsque le premier corps support est inséré dans la narine gauche et que le second corps support est inséré dans la narine droite.

5. Bouche-narine (A1, A2, A3) selon la revendication 3, dans lequel le bouche-narine inclut un premier corps support à insérer dans la narine gauche, un second corps support à insérer dans la narine droite, et une barre de liaison qui relie le premier corps support et le second corps support, et

l'unité formant soupape montée sur l'un ou l'autre des corps support est configurée pour être plus aisément séparée du corps support que l'autre unité formant soupape.

6. Bouche-narine (A1, A2, A3) selon l'une quelconque des revendications 1 à 5, dans lequel l'unité formant soupape inclut un siège de soupape formé avec une pluralité d'ouvertures de ventilation et un élément de soupape qui ferme les ouvertures de ventilation.

7. Bouche-narine (A1, A2, A3) selon la revendication 1, dans lequel l'unité formant soupape inclut un châssis tubulaire ayant ses deux extrémités ouvertes, un siège de soupape plan qui est monté sur un côté extrémité d'ouverture du châssis et est configuré pour fermer l'extrémité d'ouverture, et un élément de soupape qui est monté sur une surface latérale du siège de soupape faisant face à l'intérieur du châssis et qui est configuré pour fermer une ouverture de ventilation formée dans le siège de soupape,

sur un côté surface du siège de soupape sur lequel l'élément de soupape est agencé, un bras de guidage ayant un crochet qui est en prise avec une partie en saillie formée sur une surface périphérique intérieure du châssis est agencé le long de la direction longitudinale du châssis, et le siège de soupape est configuré pour être avancé ou rétracté dans la direction longitudinale du châssis en limitant le bras de guidage avec le châssis, et

dans un état dans lequel une pression à l'intérieur de la narine atteint un niveau prédéterminé ou plus par expiration, le siège de soupape est avancé par rapport au châssis en raison de la pression pour former un espacement libérant ainsi la pression.

8. Bouche-narine (A1, A2, A3) selon la revendication 1, dans lequel le corps support est formé d'un corps en forme de tige qui est configuré pour être inséré dans un trou traversant formé dans une cloison nasale, et a une partie d'extrémité de celui-ci agencé dans l'intérieur de la narine gauche et l'autre partie d'extrémité de celui-ci agencé dans l'intérieur de la narine droite, et

l'unité formant soupape inclut un siège de soupape formé d'un corps élastique monté sur une partie d'extrémité du corps support et un élément de soupape qui est configuré pour fermer une ouverture de ventilation formée dans le siège de soupape, et

dans un état dans lequel une pression à l'intérieur de la narine atteint un niveau prédéterminé ou plus par expiration, le siège de soupape est configuré pour être déformé de façon élastique en raison de la pression pour former un espacement entre le siège de soupape et la narine libérant ainsi la pression.

Fig.1

12

A1

14b

14c

14d

13

14b

14c

14d

11

Fig.2

A1

11

15

15

15

12

## Fig.3

A1

12

11

14b

14c

14d

14b

14c

14d

13

## Fig.4

A1

11

12

14b

14b

15

15

13

EP 1 917 993 B1

# Fig.5

# Fig.6

17

Fig.7

Fig.8

Fig.9

12

11

A2

Fig.10

24

24b (24b-1)

24c

21

## Fig.11

A2

25c

25

24 { 24c
24a

24b { 24b-2
24b-1

21b

21

## Fig.12

24

24b (24b-1)

24c′

21

Fig.13

Fig.14

**Fig.15**

**Fig.16**

## Fig.17

A2′

24c

24a

24b-1

26

21′

25

24b-2

27

## Fig.18

A2′

21′

Fig.19 (not part of the invention)

44
44c
44a
44b
46
21'
27

Fig.20 (not part of the invention)

A2

24e
24c
24a
24 { 24b { 24b-2
           24b-1
25
21b
21

## Fig.21 *(not part of the invention)*

21e

24 { 24c
       24a
       24b { 24b-2
              24b-1

25

21b

21

## Fig.22

21

24

21

24

23

## Fig.23

## Fig.24

Fig.25

Fig.26

Fig.27

Fig.28

Fig.29

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US P5259378 A
- US P4538607 A
- JP 9239047 A
- US 3884223 A
- US 20040255947 A1

### Non-patent literature cited in the description

- **STEWART DS ; RIEGER WJ.** A Device for the Management of Velopharyngeal Incompetence. *Journal of Medical Speech-Language Pathology,* 1994, vol. 2 (2), 149-155